# EUROPEAN PATENT APPLICATION

(11) **EP 2 774 636 A2**
(43) Date of publication of application: **10.09.2014**
(21) Application number: 14155857.7
(22) Date of filing: 20.02.2014
(51) Int. Cl.: A61M 5/142

(54) **Drug infusion pump**

(30) Priority: 08.03.2013 KR 20130024923
(71) Applicant: Samsung Electronics Co., Ltd., Suwon-si Gyeonggi-do 443-742 (KR); SNU R&DB Foundation, Seoul 151-742 (KR)
(72) Inventor: Lee, Sang-Hun, Suwon-si 443-742 Seoul (KR); Kim, Hee-Chan, 151-742 Seoul (KR); Lee, Jung-Chan, 151-742 Seoul (KR); Lee, Sa-Ram, 151-742 Seoul (KR)
(74) Representative: Grünecker, Kinkeldey, Stockmair & Schwanhäusser

(57) **Abstract**

A drug infusion pump is provided. The drug infusion pump includes a main body including a drive motor and a processor, and a cartridge module including a pump module and a drug cartridge and detachably provided in the main body. When the cartridge module is fitted in the main body, the pump module and the drug cartridge are accommodated in the main body and the pump module is coupled to the drive motor. The drug infusion pump is configured such that the pump module is coupled with the drive motor when the cartridge module is fitted in the main body. Accordingly, the cartridge may be easily separated and replaced so as to address a problem of a patient's inconvenience in using the drug infusion pump.

## Description

### TECHNICAL FIELD

The present disclosure relates to a drug infuser such as a syringe. More particularly, the present disclosure relates to a drug infusion pump for a diabetic patient who requires continuous maintenance and control of blood sugar.

### BACKGROUND

Typically, a diabetic patient should continuously measure blood sugar and inject insulin as needed because insulin is not normally secreted in the body. Recently, portable drug infusers have been distributed so that a diabetic patient may inject insulin by himself/herself. As for such a drug infuser, an insulin pen or an insulin pump may be exemplified.

A configuration of an insulin pen is disclosed in Korean Patent No. 1,124,194 registered on February 29, 2012 (International Publication No. WO 2004/082748 published on September 30, 2004), or the like. In general, such an insulin pen has a dial sleeve on which drug injection amounts are indicated. Thus, a user may determine an injection amount by himself/herself.

Depending on an operation type, such an insulin pump may be classified into a motorized syringe plunger type or a type in which a solenoid or a step motor and a ratchet gear are combined.

The motorized syringe plunger type insulin pump includes a plunger and a lead screw which are joined together so as to eject a drug introduced into a syringe. At this time, the patient should directly introduce an optimum dose of the drug into the syringe and then join the drug-introduced syringe with a main body of the insulin pump, specifically, the lead screw. The motorized syringe plunger type is advantageous in that the patient may easily understand the operation method and easily confirm the residual quantity of the drug due to the simple configuration thereof. However, it is inconvenient for the patient to directly introduce the drug into the syringe and to readjust the position of the plunge in order to join the syringe with the lead screw. Further, it is necessary to secure a sufficient length for the lead screw to cover the length of the syringe, i.e. a distance in which the plunger should be moved. Thus, it is unavoidable that the capacity of a mountable syringe is limited as compared to the size thereof.

An insulin pump of the type in which a solenoid or a step motor and a ratchet gear are combined is disclosed in U.S. Patent No. 4,562,751 (issued on January 07, 1986), U.S. Patent No. 4,678,408 (issued on January 07, 1987), or the like. An insulin pump of a type that uses a ratchet gear or the like is a type in which a plunger is moved forward by rotating a ratchet gear in only one direction using a displacement of a solenoid or a step motor and is similar to the motorized syringe plunger type insulin pump, except for the driving method. Accordingly, as compared with the motorized syringe plunger type insulin pump, the capacity of the syringe may be somewhat increased since no lead screw is used. However, the problem of a patient's inconvenience of having to introduce the drug into the syringe and mount the syringe is not yet addressed, even by the insulin pump of the type in which the solenoid or the step motor and the ratchet gear are combined.

Beyond the above-described types, there has been proposed an insulin pump in which a spring cartridge and a valve control mechanism are combined so as to eject a predetermined dose regardless of temperature and pressure conditions, or the like. However, the insulin pump using such a valve control mechanism has a problem in that, due to the complicated configuration, the manufacturing and purchasing costs are increased. In addition, the insulin pump has a disadvantage in that it does not yet address the problem of the user's inconvenience in use.

The above information is presented as background information only to assist with an understanding of the present disclosure. No determination has been made, and no assertion is made, as to whether any of the above might be applicable as prior art with regard to the present disclosure.

### SUMMARY

Aspects of the present disclosure are to address at least the above-mentioned problems and/or disadvantages and to provide at least the advantages described below. Accordingly, an aspect of the present disclosure is to provide a drug infusion pump having a configuration in which a consumable component may be easily replaced.

Another aspect of the present disclosure is to provide a drug infusion pump which may reduce the manufacturing and purchasing costs while allowing a consumable component to be easily replaced.

In accordance with an aspect of the present disclosure, a drug infusion pump is provided. The drug infusion pump includes a main body including a drive motor and a processor, and a cartridge module including a pump module and a drug cartridge and detachably provided in the main body. When the cartridge module is mounted in the main body, the pump module and the drug cartridge are accommodated in the main body and the pump module is coupled to the drive motor.

Other aspects, advantages, and salient features of the disclosure will become apparent to those skilled in the art from the following detailed description, which, taken in conjunction with the annexed drawings, discloses various embodiments of the present disclosure.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a perspective view illustrating a drug infusion pump according to an embodiment of the present disclosure;
FIG. 2 is a perspective view illustrating the drug infusion pump of FIG. 1 viewed in a different direction according to an embodiment of the present disclosure;
FIG. 3 is an exploded perspective view illustrating the drug infusion pump of FIG. 1 according to an embodiment of the present disclosure;
FIG. 4 is a perspective view illustrating a cartridge for the drug infusion pump of FIG. 1 according to an embodiment of the present disclosure;
FIG. 5 is a block diagram illustrating the drug infusion pump of FIG. 1 according to an embodiment of the present disclosure;
FIG. 6 is a perspective view illustrating a state where the pump module illustrated in FIG. 4 is assembled to a drive motor according to an embodiment of the present disclosure;
FIG. 7 is a perspective view illustrating an internal configuration of the pump module illustrated in FIG. 6 according to an embodiment of the present disclosure;
FIGS. 8 and 9 are views for describing an operation of the pump module illustrated in FIG. 6 according to an embodiment of the present disclosure;
FIG. 10 is a perspective view illustrating a modified embodiment of the pump module illustrated in FIGS. 6 to 9 according to an embodiment of the present disclosure;
FIG. 11 is a perspective view illustrating an internal configuration of the pump module illustrated in FIG. 10 according to an embodiment of the present disclosure;
FIGS. 12, 13, 14, and 15 are views illustrating operations of the pump module illustrated in FIG. 10 in sequence according to an embodiment of the present disclosure; and
FIGS. 16, 17, and 18 are views illustrating operations of an external gear pump as a modified embodiment of the pump module illustrated in FIG. 10 in sequence according to an embodiment of the present disclosure.

Throughout the drawings, it should be noted that like reference numbers are used to depict the same or similar elements, features, and structures.

### DETAILED DESCRIPTION

The following description with reference to the accompanying drawings is provided to assist in a comprehensive understanding of various embodiments of the present disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the present disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the present disclosure. Accordingly, it should be apparent to those skilled in the art that the following description of various embodiments of the present disclosure is provided for illustration purpose only and not for the purpose of limiting the present disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

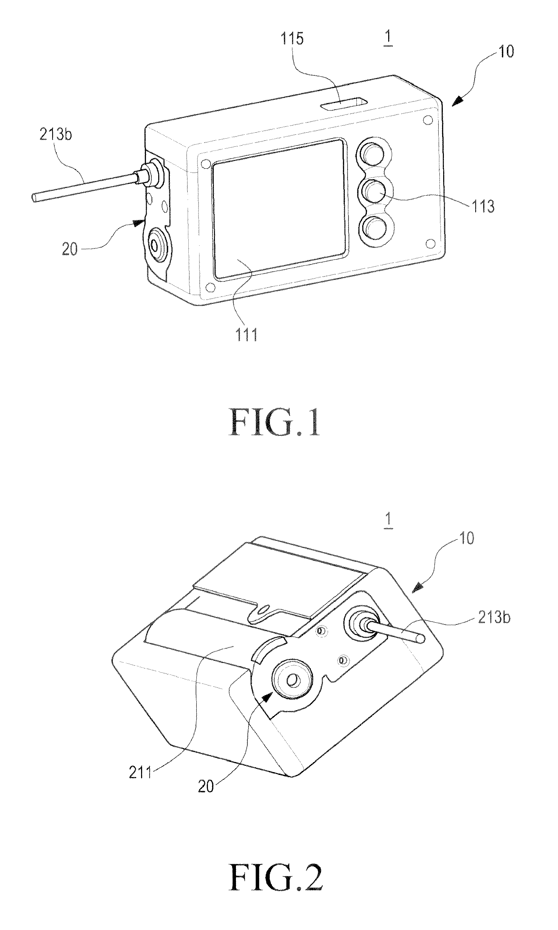

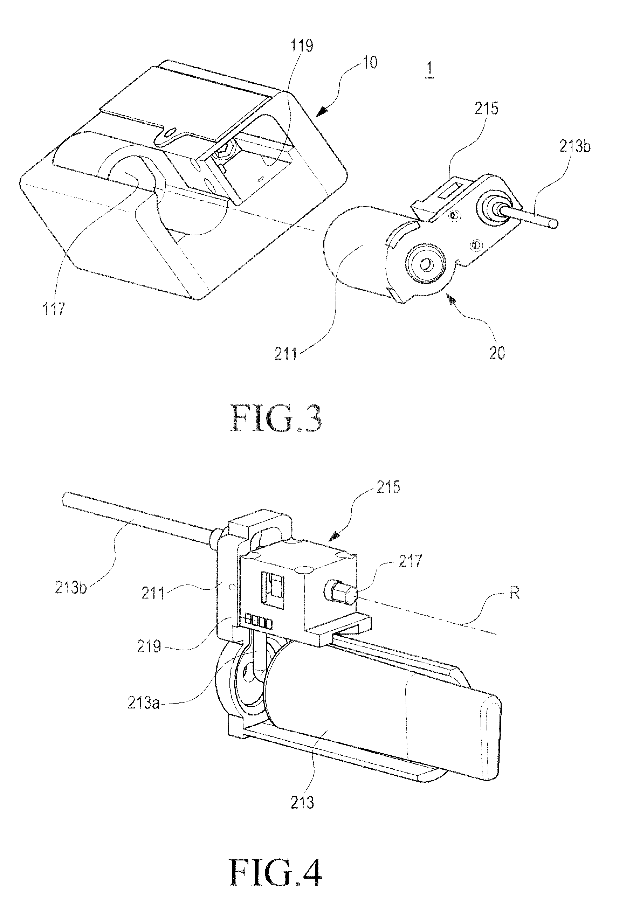

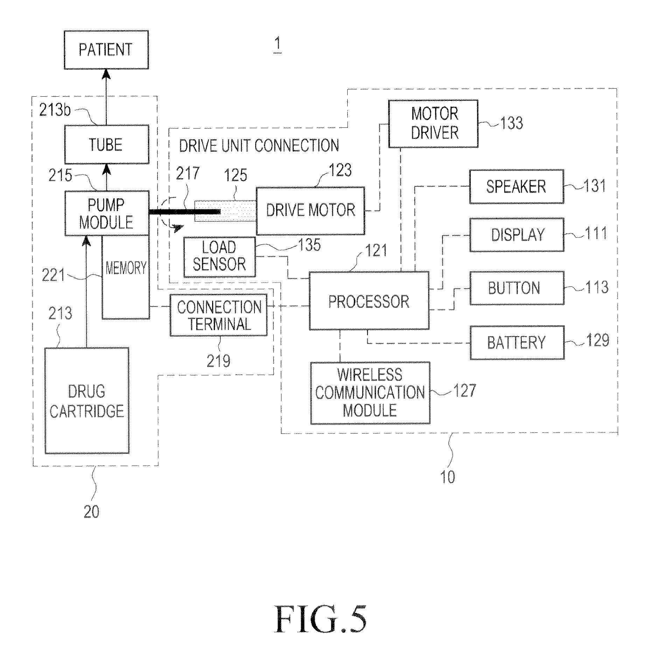

FIG. 1 is a perspective view illustrating a drug infusion pump according to an embodiment of the present disclosure, FIG. 2 is a perspective view illustrating the drug infusion pump of FIG. 1 viewed in a different direction according to an embodiment of the present disclosure, FIG. 3 is an exploded perspective view illustrating the drug infusion pump of FIG. 1 according to an embodiment of the present disclosure, FIG. 4 is a perspective view illustrating a cartridge for the drug infusion pump of FIG. 1 according to an embodiment of the present disclosure, and FIG. 5 is a block diagram illustrating the drug infusion pump of FIG. 1 according to an embodiment of the present disclosure.

As illustrated in FIGS. 1 to 5, a drug infusion pump 1 includes a main body 10, and a cartridge module 20 detachably provided in the main body 10. Here, the cartridge module 20 includes a pump module 215 and a drug cartridge 213 and the main body 10 includes a drive motor 123 that drives the pump module 215, and a processor 121 that controls the drug infusion pump 1.

The main body 10 includes a display 111 and buttons 113 provided on a front surface, and a connector 115 provided on one side surface so as to charge a battery 129 embedded therein. In addition, the drug infusion pump 1 may store information such as an infusion amount, weather information such as temperature/moisture, or the like at the time of infusing a drug or the like, and the information stored thereby may be transmitted to a personal computer or the like through the connector 115. The display 111 and the buttons 113 are used as an output device and an input device in operating the drug infusion pump 1, respectively. Another side surface of the main body 10 is formed with accommodation grooves 117 and 119 that accommodate the cartridge module 20. In an embodiment of the present disclosure, the accommodation grooves 117 and 119 are configured to independently accommodate the pump module 215 and the drug cartridge 213 of the cartridge module 20, respectively.

The main body 10 may include the drive motor 123, a coupler 125, a wireless communication module 127, a speaker 131, a motor driver 133, a load sensor 135, etc.

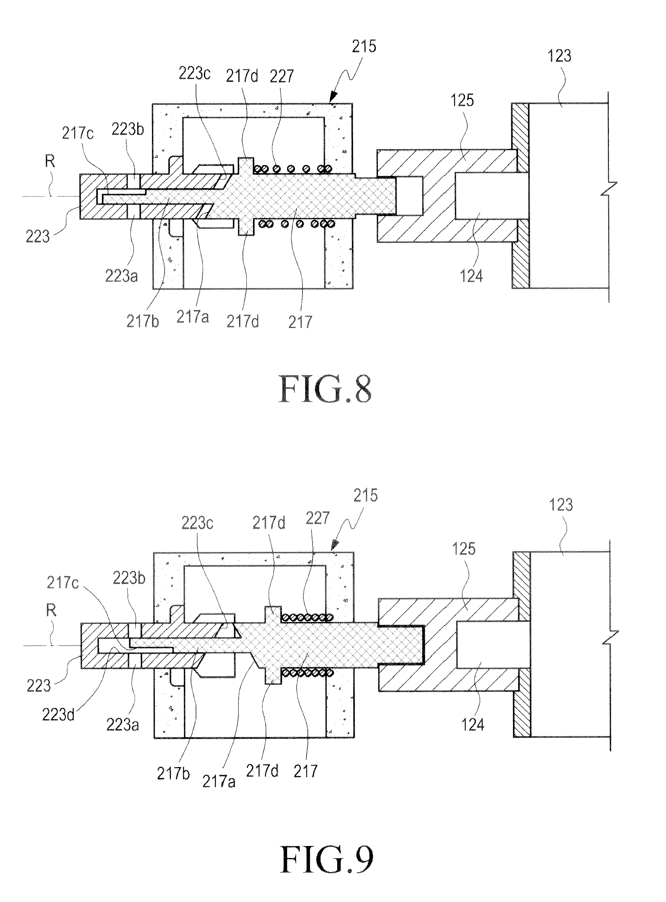

The drive motor 123 is provided to drive the pump module 215 and may be configured by a driving device that controls a driving displacement, for example, a servo motor of a solenoid or the like. A driven shaft 217 of the pump module 215 is coupled to a driving shaft 124 (see FIG. 8) of the drive motor 123 in which the driven shaft 217 is coupled to the driving shaft 124 through the coupler 125. Upon being coupled to the driving shaft 124, the driven shaft 217 may be rotated together with the driving shaft 124 about the same rotation axis R and may also rectilinearly reciprocate along the direction of the rotating axis R. It is exemplified that the driven shaft 217 is configured to be coupled to the driving shaft 124 through the coupler 125. However, a separate speed-reducing module may be provided between the driving shaft 124 and the driven shaft 217.

The wireless communication module 127 enables the drug infusion pump 1 to be connected with a portable communication device such as a mobile communication terminal, a personal computer, a blood sugar sensor, or the like. When the wireless communication module 127 is connected to the blood sugar sensor, it is possible to determine whether to infuse a drug and adjust an infusion amount of the drug depending on a patient's blood sugar detected by the blood sugar sensor. In addition, the information such as the level detected by the blood sugar sensor, the infusion amount at the time of drug infusion, or the like may be stored in the personal computer or the portable communication device or transmitted to the patient's physician in real time. Furthermore, the patient's physician may input and transmit a new prescription as needed with reference to the received disease information of the patient, the drug infusion amount, environmental information, or the like.

The battery 129 supplies power for operating the drug infusion pump 1 and may be charged through the connector 115. The speaker 131 may generate a sound signal such as an alarm sound or output a sound that guides or instructs a using method according to the operation of the drug infusion pump 1 step by step. The motor driver 133 generates a driving signal that operates the drive motor 123 according to a previously input program and a control of the processor 121.

The load sensor 135 detects an abnormal operation phenomenon such as narrowness or blockage of a drug flow passage in the process of operating the drug infusion pump 1 by sensing a variation of load applied to the driving shaft 124 or the drive motor 123. As for the load sensor 135, an optical sensor such as a hall effect sensor or a photo sensor may be used. When the load sensor 135 detects the abnormal operation phenomenon from the driving shaft 124 or the drive motor 123, the processor 121 performs a control in such a manner that the operation of the drive motor 123 is stopped and the alarm signal or the like is generated.

The drug cartridge 213 of the cartridge module 20 stores a drug, for example, insulin, and may be made of an elastic material. The drug cartridge 213 made of the elastic material compresses the stored drug constantly. Accordingly, when the pump module 215 is operated, the drug stored in the drug cartridge 213 may be smoothly supplied to the pump module 215. Of course, even if the drug cartridge 213 is not made of the elastic material, the drug stored in the drug cartridge 213 may be compressed using a mechanical structure. For example, a plunger and a spring used in an ordinary syringe structure may be arranged inside the drug cartridge 213 so as to compress the drug stored in the drug cartridge 213. In addition, an elastically deformable diaphragm structure may be installed inside the drug cartridge 213 to divide the internal space of the drug cartridge 213 into a gas chamber and a drug chamber. In such a case, when the gas chamber is filled with a gas so that the internal space of the drug cartridge 213 may be fully occupied by the gas chamber and then, a drug is introduced into the drug chamber, the drug introduced into the drug chamber within the drug cartridge 213 may be compressed by the pressure of the gas chamber. Such a diaphragm structure may be made of an autonomously elastically deformable film material or may be configured using a bellows structure.

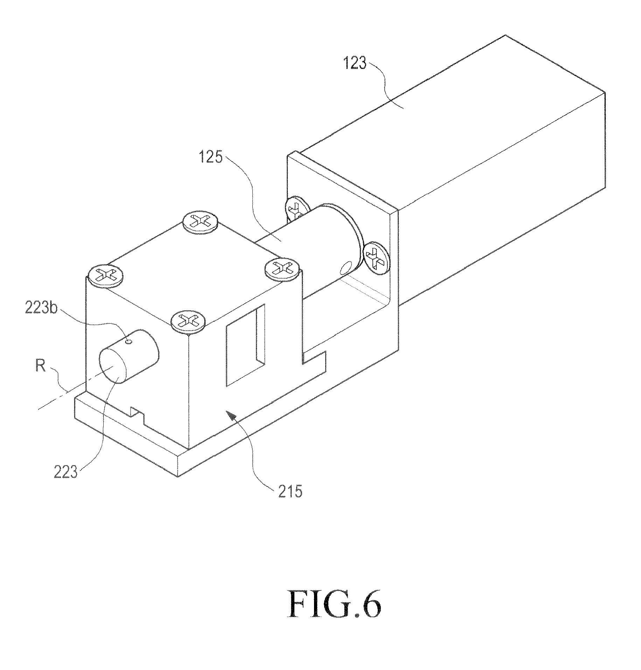

The drug cartridge 213 is connected to the pump module 215 through a supply tube 213a. In addition, the cartridge module 20 is provided with an injection tube 213b connected to the pump module 215. The injection tube 213b is connected to an outlet port 223b (see FIG. 6) of the pump module 215 so that the drug ejected according to the operation of the pump module 215 is supplied to the patient.

The pump module 215 of the cartridge module 20 may further include a memory 221 and a connection terminal 219. The memory 221 may be configured by a read-only memory or a read/write memory. When only the information that may be input in the process of manufacturing a drug, for example, the expiration date of the drug, a genuine product certification code, a driving condition of a drive motor or the like, is stored in the drug cartridge, the memory 221 may be configured by a read-only memory. In addition, when the memory 221 is configured by a read/write memory, a physician's prescription may be input or renewed according to a patient's disease. Even if the memory 221 is configured by the read/write memory, the information input in the process of manufacturing the drug may be set to be unchangeable.

The connection terminal 219 is connected with the memory 221 so as to connect the memory 221 to the processor 121. Accordingly, when the cartridge module 20 is fitted in the main body 10, the processor 121 may read the information stored in the memory 221 and set a driving condition of the driving motor 123 according to the information stored in the memory 221.

The cartridge module 20 may be provided with a case member 211 so as to configure the pump module 215 and the drug cartridge 213 as a single module. That is, the pump module 215 and the drug cartridge 213 are fixed to the case member 211. The case member 211 may be provided so as to at least partly enclose the drug cartridge 213 and be at least partly exposed to the outside in a state where the cartridge module 20 is fitted in the main body 10. In an embodiment, each of the drug cartridge 213 and the case member 211 is at least partly made of a transparent material. This is to allow a patient to readily confirm the remaining amount of the drug within the drug cartridge 213.

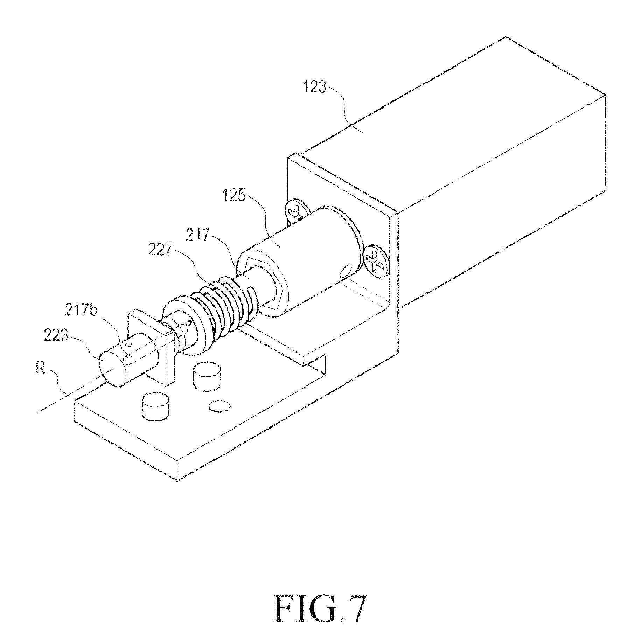

FIG. 6 is a perspective view illustrating a state where the pump module illustrated in FIG. 4 is assembled to a drive motor according to an embodiment of the present disclosure, FIG. 7 is a perspective view illustrating an internal configuration of the pump module illustrated in FIG. 6 according to an embodiment of the present disclosure, and FIGS. 8 and 9 are views for describing an operation of the pump module illustrated in FIG. 6 according to an embodiment of the present disclosure.

Hereinafter, a configuration of the pump module 215 and the coupling structure between the pump module 215 and the drive motor 123 will be described with reference to FIGS. 6 to 9. The pump module 215 illustrated in FIGS. 6 to 9 sucks or ejects a drug by the rectilinear reciprocating movement of a piston member 217b. First, the driven shaft 217 is mounted in such a manner that it may be rotated and rectilinearly reciprocated on the pump module 215. When coupling the driving shaft 124 through the coupler 125, the driven shaft 217 is coupled to be fixed to the coupler 125 in the rotation direction and to be capable of rectilinearly reciprocating in the rotating axis R direction.

The pump module 215 is provided with a liner 223 fixed therein together with the driven shaft 217. The liner 223 includes a chamber 223d which extends in the rotating axis R direction. The liner 223 is opened at one end. The liner 223 includes an inlet port 223a and an outlet port 223b, and a part of the liner 223 may protrude to the outside of the pump module 215. When a part of the liner 223 protrudes to the outside of the pump module 215, the inlet and outlet ports 223a and 223b may be arranged to be exposed to the outside of the pump module 215. The inlet port 223a is connected to the drug cartridge 213 through the supply tube 213a, and the injection tube 213b is connected to the outlet port 223b as described above.

An end of the driven shaft 217 is coupled to the coupler 125. In general, the driven shaft 217 has a circular cross-section to be rotatably disposed in the pump module 215. However, it is desirable that a part of the circumferential surface of the driven shaft 214 is formed to be flat at a portion where the driven shaft 214 is coupled to the coupler 125. This is to enable the driven shaft 217 be fixed to the coupler 125 in the rotation direction while being rotatable within the pump module 215.

The piston member 217b is provided at the other end of the driven shaft 217. The piston member 217b extends in the rotating axis R direction from the other end of the driven shaft 217 and is accommodated within the chamber 223d of the liner 223. The piston member 217b is rotated within the chamber 223d together with the driven shaft 217 as well as rectilinearly reciprocated in the rotating axis R direction. At the end of the piston member 217b, a notch 217c is formed. The inlet port 223a and the outlet port 223b are not connected with the chamber 223d in general by being closed by the outer circumferential surface of the piston member 217b but may be connected to the chamber 223d depending on the rotating position of the piston member 217b. That is, depending on the rotation of the piston member 217b, the notch 217c alternately connects the inlet port 223a and the outlet port 223b to the chamber 223d.

The driven shaft 217 that rotates in a section where the inlet port 223a is connected to the chamber 223d is moved in a direction away from the liner 223, that is, in a direction where the piston member 217a is released from the chamber 223d. At this time, the outlet port 223b is closed by the outer circumferential surface of the piston member 217b. Accordingly, the drug stored in the drug cartridge 213 flows into the inside of the chamber 223d of which the pressure has been reduced. The piston member 217b that rotates in a section where the outlet port 223b is connected to the chamber 223d is rectilinearly moved in a direction of approaching the closed end of the liner 223, that is, in the direction in which the piston member 217b is further introduced into the chamber 223d. At this time, the inlet port 223a is closed by the outer circumferential surface of the piston member 217b. Accordingly, the internal pressure of the chamber 223d is increased and the drug flown into the inside of the chamber 223d is ejected through the outlet port 223b.

In order to convert the rotation of the piston member 217b into a rectilinear reciprocating movement, the pump module 215 is provided with an elastic member 227 and inclined surfaces 217a and 223c. The elastic member 227 will provide an elastic force acting on the driven shaft 217 so as to bias the driven shaft 217 toward the liner 223 within the pump module 215. On the outer peripheral surface of the driven shaft 217, a support rib 217d is formed to support one end of the elastic member 227. The support rib 217d may be formed in a ring shape that extends around the circumference of the driven shaft 217. The inclined surfaces are composed of a first inclined surface 217a formed at the other end of the driven shaft 217, and a second inclined surface 223c formed at the opened end of the liner 223. The first and second inclined surfaces 217a and 223c are formed to be inclined in relation to the rotating axis R, for example at the same angle in relation to the rotating axis R. By the arrangement of the elastic member 227 and the inclined surfaces 217a, 223c, the driven shaft 217 is rectilinearly reciprocated within the pump module 215 while being rotated together with the driving shaft 124. As illustrated in FIG. 8, at the position where the first and second inclined surfaces 217a and 223c are aligned in the same direction, the driven shaft 217 is moved forward to the liner 223 by the elastic force of the elastic member 227, thereby forcing the first and second inclined surfaces 217a and 223c to come in close contact with each other. When the driven shaft 217 is rotated by an angle of 180 degrees from the position illustrated in FIG. 8, the first and second inclined surfaces 217a and 223c are aligned to be inclined in the reversed directions in relation to each other. Accordingly, as illustrated in FIG. 9, the driven shaft 217 is moved away from the liner 223. Consequently, the driven shaft 217 is rectilinearly reciprocated between the positions illustrated in FIGS. 8 and 9 while being rotated together with the driving shaft 124.

As the driven shaft 217 is rectilinearly reciprocated while being rotated, the piston member 217b is also rotated and rectilinearly reciprocated within the chamber 223d.

Referring to FIG. 8, in a state where the piston member 217b has been fully introduced into the chamber 223d or while the piston member 217b is being introduced into the chamber 223d, the inlet port 223a is closed and the outlet port 223b is opened by the notch 217c. Accordingly, the drug within the chamber 223d is administrated to a patient through the outlet port 223b and the injection tube 213b. In a state where the piston member 217b has been moved in the direction of being released from the chamber 223d as illustrated in FIG. 9 or while the piston member 217b is being moved in the direction of being released from the chamber 223d, the outlet port 223b is closed and the inlet port 223a is opened by the notch 217c. At this time, the internal pressure of the chamber 223d decreases and thus, the drug stored in the drug cartridge 213 is introduced into the chamber 223d.

Consequently, the piston member 217b performs while being rotated and rectilinearly reciprocated within the chamber 223d and the internal pressure of the chamber 223d increases or decreases. Accordingly, the drug stored in the drug cartridge 213 is alternately introduced into the chamber 223d through the first passage, i.e. the supply tube 213a and ejected from the chamber 223d through the second passage, i.e. the injection tube 213b.

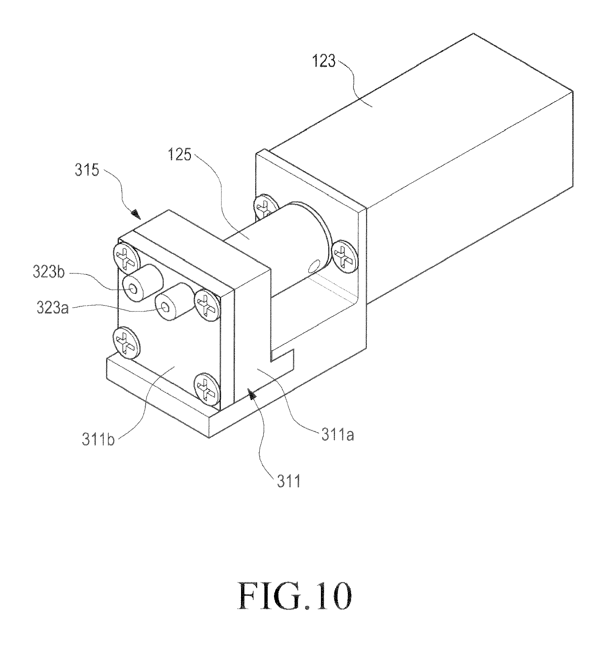

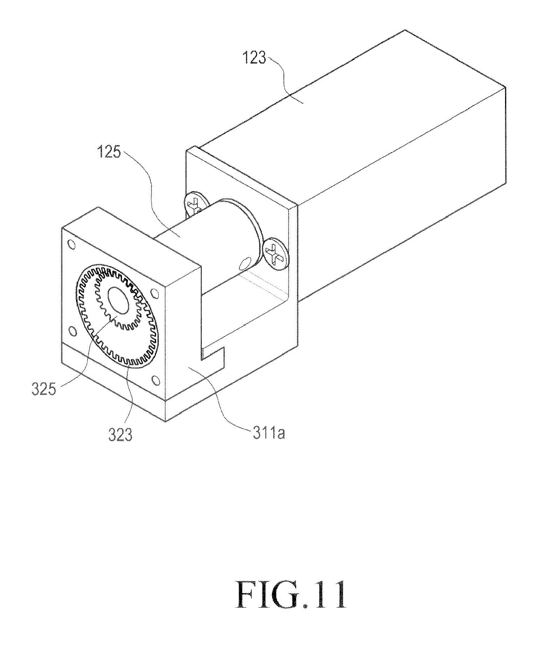

FIG. 10 is a perspective view illustrating a modified embodiment of the pump module illustrated in FIGS. 6 to 9 according to an embodiment of the present disclosure. FIG. 11 is a perspective view illustrating an internal configuration of the pump module illustrated in FIG. 10 according to an embodiment of the present disclosure.

Referring to FIGS. 10 and 11, the pump module 315 employs a gear pump. This is in contrast to the pump module 215 illustrated in FIG. 6 that performs a pumping operation by converting the rotating movement of the driving shaft 124 into the rectilinear reciprocating movement of the piston member 217b.

The pump module 315 includes a pair of gears 323 and 325 accommodated within the casing 311 which is formed by assembling a main casing 311a and an auxiliary casing 311b. One of the main casing 311 a and the auxiliary casing 311b is provided with inlet/outlet ports 323a and 323b so as to allow the drug to be introduced or ejected. In the structure illustrated in FIG. 10, a configuration where the inlet/outlet ports 323a and 323b are arranged in the auxiliary casing 311b is exemplified. Within the casing 311, a pair of gears 323 and 325 are arranged to be engaged with each other. Of the gears, at a position where the teeth of the first gear 323 are disengaged from the teeth of the second gear 325 (hereinafter, the position will be referred to as a "first position"), the inlet port 323a is connected to the internal space of the casing 311, and at the position where the teeth of the first gear 323a are engaged with the teeth of the second gear 325 again (hereinafter, the position is referred to as a "second position"), the outlet port 323b is connected to the internal space of the casing 311. Accordingly, at the first position, the drug flows into each space between the teeth of the first and second gears 323 and 325 through the inlet port 323a, and, at the second position, the drug flown into each space between the teeth of the first and second gears 323 and 325 is forcibly ejected through the outlet port 323b. The gear pump as described above is configured by an internal gear pump or an external gear pump.

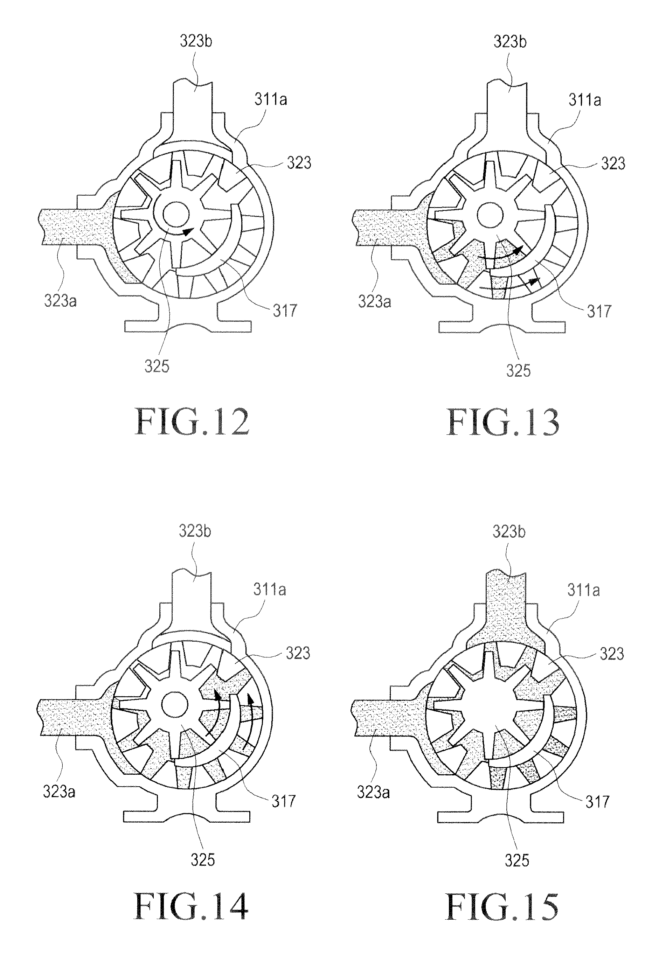

FIGS. 12 to 15 are views illustrating operations of the pump module illustrated in FIG. 10 in sequence according to an embodiment of the present disclosure.

Referring to FIGS. 12 to 15, the first gear 323 includes gear teeth formed on the inner periphery thereof and the second gear 325 has gear teeth formed on the outer periphery thereof. The first gear 323 is accommodated within the casing 311 to be engaged with the second gear 325 to be rotated, and the second gear 325 is engaged with the driven shaft 217 to be rotated by the operation of the drive motor 123. In an embodiment, the inner diameter of the first gear 323 is larger than the outer diameter of the second gear 325. In a region where the teeth of the first and second gears 323 and 325 are not engaged in the internal space of the casing 311, a diaphragm 317 is disposed. The inlet port 323a and the outlet port 323b are maintained in a state where they are substantially spaced apart from each other by the diaphragm 317. The diaphragm 317 may be formed on an inner wall of one of the main casing 311 a and the auxiliary casing 311b. Since the diaphragm 317 is disposed, the feeding of the drug from the inlet port 323a to the outlet port 323b is enabled only by the rotation of the first and second gears 323 and 325.

In the configuration illustrated in FIGS. 12 to 15, each of the first and second gears 323 and 325 is rotated counterclockwise and, according to the rotation of the first and second gears 323 and 325, the drug flown into the inlet port 323a flows into the gaps between the teeth of the first and second gears 323 and 325 to be ejected to the outlet port 323b via the diaphragm 317. At this time, at the first position, the teeth of the first gear 323 are disengaged from the teeth of the second gear 325, thereby expanding the gaps between the teeth. By the expanded extent of the gaps between the teeth, the pressure is also lowered and thus, the drug is forced to flow into the gaps. At this time, when the drug cartridge 213 is fabricated from an elastic material and compresses the drug stored therein, the flow of the drug into the pump module 315 will be further activated. At the second position, the teeth of the first gear 323 are engaged with the teeth of the second gear 325 again. Accordingly, at the second position, the drug is forcibly ejected from the gaps between the teeth to flow to the injection tube 213b through the outlet port 323b.

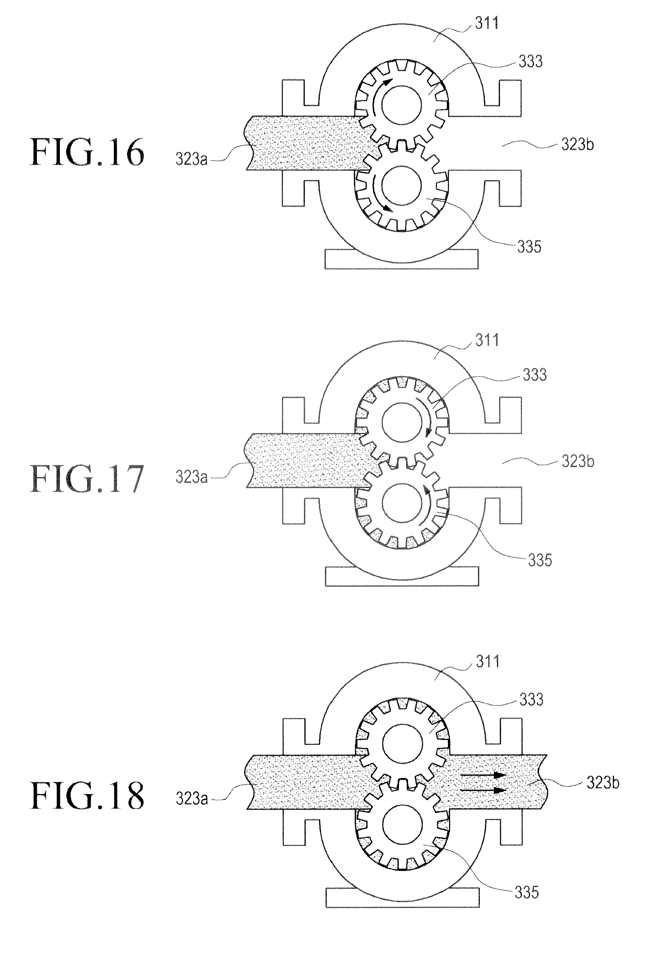

FIGS. 16 to 18 are views illustrating operations of an external gear pump as a modified embodiment of the pump module illustrated in FIG. 10 in sequence according to an embodiment of the present disclosure.

Referring to FIGS. 16 to 18, the external gear pump is configured such that a pair of sun gears 333 and 335 are engaged with each other. Of the gears, the first gear 333 is engaged with the driven shaft 217 to be rotated, and the second gear 335 is engaged with the first gear 333 to be rotated. At the first position, i.e., at a position adjacent to the inlet port 323a, the teeth of the first gear 333 are disengaged from the teeth of the second gear 335. Thus, the drug flows into the inside of the casing 311 through the inlet port 323a. The drug flown into the casing 311 is accommodated in each gap between the teeth of the first and second gears 333 and 335, and as the first and second gears 333 and 335 are rotated, the drug is fed to the second position adjacent to the outlet port 323b. At the second position, the teeth of the first gear 333 are engaged with the teeth of the second gear 335 again. Thus, the drug between the teeth is forcibly ejected to flow to the injection tube 213b through the outlet port 323b.

Upon comparing with a motorized syringe plunger type and the combined type of a solenoid or a step motor with a ratchet gear of the related art, the drug infusion pump according to the present disclosure may reduce a driving space by configuring the pump module using a piston member or gears. Accordingly, with the same size, the drug infusion pump according to the present disclosure may further increase the drug storage volume as compared with pumps of the related art. In addition, the configuration of the cartridge module which is a consumable component may be simplified. Accordingly, the user's or patient's economic burden according to the replacement of the cartridge module may be reduced. Moreover, a process of adjusting the position of the lead screw separately is not required in mounting the cartridge module in the main body of the drug infusion pump, and the cartridge module may be easily mounted in the accommodation groove provided to be suitable for the cartridge module. Accordingly, the user may use the drug infusion pump easily and conveniently.

The drug infusion pump configured as described above has an advantage in that, since the drug infusion pump is configured such that the pump module is assembled with the drive motor only by fitting the cartridge module in the main body, it is easy to disassemble and replace the consumable component, i.e., the cartridge module. In addition, since the drug cartridge is packed in a sealed and sterilized state to be supplied to a user or patient, the drug infusion pump may be instantly used only by replacing the cartridge module, and since it is not required to adjust the position of the plunger or the like, the problem of the user's inconvenience is addressed. In addition, since the pump module may be driven without using a lead screw, the pump module may be miniaturized so that the volume of the drug cartridge may be increased. Furthermore, there is an advantage in that, since drug infusion information or the like may be input in the process of manufacturing or input according to a prescription by a physician or a pharmacist using a memory equipped in the cartridge module, it is not necessary for the patient to adjust the injection amount or the like. Moreover, there is an advantage in that, since the drug infusion pump may be connected with a blood sugar sensor or a portable communication device through a wireless communication module, the patient's condition or the like at the time of injection may be stored or transmitted to the physician so as to monitor the patient's condition.

While the present disclosure has been shown and described with reference to various embodiments thereof, it will be understood by those skilled in the art that various changes in form and details may be made therein without departing from the spirit and scope of the present disclosure as defined by the appended claims and their equivalents.

## Claims

1. A drug infusion pump comprising:
a main body including a drive motor and a processor; and
a cartridge module including a pump module and a drug cartridge, the cartridge module detachably provided in the main body,
wherein, when the cartridge module is fitted in the main body, the pump module and the drug cartridge are accommodated in the main body and the pump module is coupled to the drive motor.

2. The drug infusion pump of claim 1, wherein the cartridge module further includes a memory and a connection terminal that connects the memory to the processor.

3. The drug infusion pump of claim 2, wherein the memory is a read-only memory that stores at least one of an identification code of the cartridge module, an expiration date of a drug stored in the drug cartridge, a genuine product certification code, and a driving condition of the drive motor.

4. The drug infusion pump of claim 3, wherein, when the cartridge module is fitted in the main body, the processor reads information stored in the memory to set the driving condition of the drive motor.

5. The drug infusion pump of any one of claims 2 through 4, wherein the memory is a read/write memory where a prescription according to a patient's disease may be input or renewed.

6. The drug infusion pump of claim 5, wherein, when the cartridge module is fitted in the main body, the processor reads information stored in the memory to set the driving condition of the drive motor.

7. The drug infusion pump of any one of claims 1 through 6, wherein the drive motor includes a coupler fixed to a driving shaft and the pump module includes a driven shaft which may be coupled to the coupler, and
the driven shaft is coupled to the coupler to be rotated together with the driving shaft.

8. The drug infusion pump of claim 7, wherein the pump module further includes:
a liner that provides a chamber extending in a direction of a rotating axis of the driven shaft; and
a piston member extending from an end of the driven shaft and accommodated in the chamber,
wherein, when the driven shaft is rotated together with the driving shaft, the piston member is rectilinearly reciprocated in the direction of the rotating axis within the chamber.

9. The drug infusion pump of claim 8, wherein the pump module further includes:
a first inclined surface provided at the end of the driven shaft and formed to be inclined in relation to the rotating axis;
a second inclined surface provided at an end of the liner and formed to correspond to the first inclined surface; and
an elastic member that provides an elastic force in a direction of forcing the first and second inclined surfaces to come in close contact with each other,
wherein, when the driven shaft is rotated, the first inclined surface is rotated in a state where it faces the second inclined surface so that the driven shaft and the piston member are rectilinearly reciprocated.

10. The drug infusion pump of any one of claims 8 through 9, wherein as the piston member is rectilinearly reciprocated within the chamber, the drug stored in the drug cartridge is alternately flown into the chamber through a first passage and ejected from the chamber through a second passage.

11. The drug infusion pump of any one of claims 7 through 10, wherein the pump module further includes an internal gear pump or an external gear pump that accommodates at least one pair of gears within a casing, and
wherein, of the gears, when a first gear is engaged with the driven shaft and rotated together with the driven shaft, the drug stored in the drug cartridge flows into the casing through the first passage and the drug flown into the casing is ejected through the second passage.

12. The drug infusion pump of any one of claims 1 through 11, wherein the main body further includes a wireless communication module to be connected with a body sugar sensor or a portable communication device through the wireless communication module.

13. The drug infusion pump of any one of claims 1 through 12, wherein the main body further includes a load sensor that detects a load applied to the operation of the drive motor.

14. The drug infusion pump of any one of claims 1 through 13, wherein the drug cartridge is fabricated from an elastic material to compress the drug stored therein.
